# EUROPEAN PATENT APPLICATION

(11) **EP 4 265 245 A1**
(43) Date of publication of application: **25.10.2023**
(21) Application number: 23165396.5
(22) Date of filing: 30.03.2023
(51) Int. Cl.: A61K 31/194, A61K 9/00, A61K 31/047, A61K 31/122, A61K 33/00, A61K 33/06, A61P 17/02, A61P 31/00

(54) **NEW COMPOSITION FOR USE IN THE TREATMENT OF WOUNDS**

(30) Priority: 20.04.2022 IT 202200007811
(71) Applicant: Industria Farmaceutica Nova Argentia S.r.l., 20064 Gorgonzola (IT)
(72) Inventor: RIVA, Danilo, 22033 ASSO (IT); LAFFRANCHI, Alberto, 22036 Erba (IT); PUMILIA, Gloria, 20125 Milano (IT); LEROSE, Alice, 20099 Sesto San Giovanni (IT)
(74) Representative: Valenza, Silvia

(57) **Abstract**

The use of an aqueous solution of Citric Acid Monohydrate + Sodium Bicarbonate, preferably combined with the subsequent use of a gel based on water enriched in oxygen, D-panthenol and hyaluronic acid, as a new effective treatment of skin lesions such as, for example, acute lesions, including infected ones, in particular if consequent to radiotherapy, bedsores, diabetic foot and wounds from a traumatic event, is described.

## Description

### FIELD OF THE INVENTION

The present invention relates to the field of cleansing solutions for use in the treatment of skin and/or mucous membrane lesions, in particular acute, sub-acute and chronic lesions, including infected ones.

### STATE OF THE ART

Cleansing of a wound is the first step in the dressing and is typically carried out by irrigation of saline, borated water, or iodine solution (chlorhexidine in cases of iodine allergy) followed by local application of hemostatics, antiseptics or antibiotics in powder/cream, depending on the appearance of the wound. In the event of wound with substance loss, cleansing is followed by stitches.

In the case of chronic skin lesions, the wound (surgical or non-surgical) is still cleaned, following the criteria of the so-called advanced dressings.

In the field of oncology, radiotherapy, as a single solution or in combination with chemotherapy and/or surgery, represents an important and currently irreplaceable methodology in the treatment of neoplasms. This therapeutic technique is not free of side effects, even very disabling for the patient, such as the presence of skin lesions.

Acute, sub-acute and chronic skin lesions from radiotherapy are worthy of attention. These lesions are generally treated using electromagnetic fields both for analgesic purposes and with the aim of promoting healing too. There remains the problem of cleansing open wounds which are generally cleaned with 3% Borate Water.

Boric acid can be absorbed in toxic amounts through the gastrointestinal tract, by inhalation or, in our case, through skin lesions.

Following the use of large amounts of boric acid on wounds or sores, cases of poisoning and death have occurred, especially in children. The mechanism of the toxic action is unknown and numerous organs and systems are affected, in particular skin, kidneys and the digestive tract. The toxic effects also affect the CNS and the lung, with mainly hemorrhagic lesions of obscure genesis. The main symptoms of boric acid poisoning are vomiting, diarrhea, visceral pain, skin erythema followed by desquamation, CNS stimulation followed by depression, restlessness, headache. Metabolic acidosis and severe water and salt imbalance are also common. Furthermore, boric acid in these situations can cause seizures, changes in body temperature and kidney damage which can be evidenced by oliguria.

Therefore, there is a need to replace the latter for wound cleansing with a non-toxic substance capable of also stimulating cell regeneration and preventing or treating any microbial infections, in addition to cleansing the wounds.

Citric acid is one of the standard ingredients in cosmetic formulations, precisely because of its ability to adjust the pH of the formulation; due to its ability to cleanse, it is used in biodegradable powder detergents, thus replacing phosphates (recognized pollutants).

Carbonates (bicarbonate, carbonic acid, and CO₂) are the indispensable components of the cytoplasm and all biological liquids.

The bicarbonate content of the organism is strictly conserved, and its deficiency causes cellular and tissue respiration impairment, which is followed by the development of a series of pathological states.

Common and curative drinking waters are usually bicarbonate solutions.

The object of the present invention is to provide a cleansing solution for skin lesions which is more effective than simple saline, but less toxic than borated water and iodine solution, and which stimulates the cellular energy cycle while reducing, if possible, also the sensation of pain. The object of the present invention is also to provide a product for use in the topical treatment of acute and/or chronic skin and/or mucous membrane lesions which promotes healing thereof.

### SUMMARY OF THE INVENTION

The present invention addresses the aforementioned problems by means of an aqueous solution in which a mixture of citric acid and sodium bicarbonate has been dissolved, said aqueous solution for topical use as an antimicrobial and/or in the topical treatment of acute and/or chronic skin and/or mucous membranes lesions, including infected ones.

The sodium bicarbonate present in the product converts citric acid into sodium citrate thus increasing the alkalinity of the tissue of the injured area, thus making it less irritating.

The role of bicarbonates is, however, much more than this: CO₂ is partly produced, while a portion of the carbonates (inorganic carbonates) remains *in situ* in the injured area, playing an important role in cellular respiration (a fundamental aspect in necrotic areas, typical of bedsores and diabetic foot).

To verify the efficacy of the mixtures object of the present invention, a group of patients affected by acute and chronic skin lesions was treated. Surprisingly, the aqueous composition of the invention showed significant results both in the reduction of pain associated with the lesion and in healing, also showing an antimicrobial effect, in particular against *Klebsiella, E. coli, Candida Albicans, Staphylococcus aureus* and *Pseudomonas aeruginosa.*

*In vitro* studies show that the treatment with the solution of Citric Acid + Sodium Bicarbonate according to the present invention stimulates, already in the short term, tissue repair mechanisms with evidence of:
- morphological integrity;
- inhibition of hyperkeratosis and general epidermal hypertrophy with pyknosis;
- maintenance of physiological epithelial-mesenchymal interactions;
- conservation of physiological processes of terminal differentiation;
- promotion of a rapid neo-deposition of collagen fibers and remodeling of the extracellular matrix.

One hypothesis on the rationale for the surprising results is that citric acid can intervene in the biochemistry of the Krebs cycle of the cells in the injured area and that bicarbonate supplies those indispensable components of the cell cytoplasm and biological fluids. The results obtained lead us to believe that cleansing with the solution of Citric Acid and Sodium Bicarbonate, according to the present invention, constitutes a very advantageous treatment in the majority of lesions and has the ability to thoroughly cleanse the wound, facilitating the elimination of fibrin, promoting skin regeneration processes and promoting cicatrization and lesion healing. Furthermore, we observed a favorable remodulation of the wound cicatrization process, with a clear limitation of the possible deformations of the same scars.

The object of the present invention is also a kit for sequential use in the topical treatment of skin and/or mucous membrane lesions, said kit comprising:
a solution according to the invention, to be applied first;
a gel based on water enriched in oxygen, D-Panthenol and hyaluronic acid, to be applied subsequently.

The kit has been shown to further improve the healing of skin lesions.

### DETAILED DESCRIPTION OF THE INVENTION

Preferably the aqueous composition of the invention is obtained by dissolving in water a solid mixture consisting of:

| | |
|---|---|
| Citric acid | 40-50% w/w; |
| NaHCO₃ | 50-60% w/w. |

Preferably, citric acid is monohydrate.

The water is preferably purified water or saline.

The solid mixture of Citric acid+NaHCO₃ is dissolved in an adequate amount of water to obtain an aqueous solution comprising:

| | |
|---|---|
| Citric acid | 5-35% w/w, preferably 5-20% w/w; |
| NaHCO₃ | 5-40% w/w, preferably 5-22% w/w. |

The aqueous composition of the invention has a pH of 4-5, preferably 4.5.

Preferably, the composition of the invention further comprises a hemostatic agent.

The hemostatic agent is preferably a combination of iron ammonium citrate (FAC) and potassium alum in a weight ratio of 4:1-2:1, as described in WO2021176410 by the same applicant. Preferably the hemostatic agent FAC+Potassium Alum is comprised in the composition of the invention at 2-15% w/w.

Preferably the composition of the invention also comprises a moisturizing and/or antioxidant and/or antimicrobial agent. Preferably said moisturizing and/or antioxidant and/or antimicrobial agent is a mixture of 1,2-hexanediol, 1,2-octanediol and Tropolone (commercially available as SymDiol 68T). Preferably the moisturizing and/or antioxidant and/or antimicrobial agent is contained in the composition at 0.2-2% w/w.

According to a particularly preferred embodiment, the aqueous composition of the invention comprises or consists of:

| | |
|---|---|
| Citric acid monohydrate | 5-20%, preferably 12-16%; |
| NaHCO₃ | 5-22%, preferably 14-18% |
| FAC + potassium alum 4:1-2:1 | 2-15%, preferably 5-9%; |

| | |
|---|---|
| SYMDIOL 68T | 0.2-2%, preferably 0.5-1%; |
| Water | balance to 100%; |

where the percentages are % w/w referred to the total weight of the composition.

The composition of the invention can be in the form of a solution or a spray, to obtain the aforementioned therapeutic effects it should be applied daily (1-3 times/day) on the lesion. Preferably, the solution of the invention is applied 1-3 times/day on the lesion by means of soaked gauze compresses lasting 20-30 minutes.

The rinses with the aqueous solution of Ac. Citric Acid Monohydrate + Sodium Bicarbonate, even at the highest concentrations indicated above, have proved to be useful and free of side effects in the cleansing of open wounds and fistulas of various kinds, especially if infected.

The kit according to the invention preferably further comprises a gel comprising:

| | |
|---|---|
| D-panthenol | 1-9%, preferably 4-6%; |
| Sodium hyaluronate | 0.05-4%, preferably 0.1-0.3%; |
| Kaqun water | balance to 100; |

where the percentages are % w/w referred to the total weight of the composition.

The gel preferably also comprises:

| | |
|---|---|
| a pH adjuster | 0.5-3%, preferably 1-2%; |
| a gelling agent | 0.5-3%, preferably 1-2% |
| SYMDIOL 68T | 0.2-2%, preferably 0.5-1%; |

The pH adjuster is preferably triethanolamine (TEA). The gelling agent is preferably carbomer (carbopol 940).

The composition of the invention, preferably in association with the gel, constitutes a new effective treatment of acute skin lesions, including infected ones, in particular if they result from radiotherapy, burns, bedsores, diabetic foot and wounds from a traumatic event.

It has been surprisingly observed that in infected, relapsing fistulas, present for months, in some cases years, repeatedly treated with antibiotic therapy, but always relapsing, the introduction of washings with the solution of Citric Acid and Sodium Bicarbonate according to the present invention leads to lesion healing within 4 weeks, on average.

The solution object of the present invention is capable of stimulating the healing of infected wounds, regardless of their cause and regardless of antibiotic therapy. The composition of the invention shows effectiveness for the treatment of acute skin lesions, including infected ones, in particular if resulting from radiotherapy, bedsores, diabetic foot and wounds from a traumatic event, simple skin abrasions, cleansing of acute and sub-acute radiodermatitis, oral cavity, genital and anal mucositis, for the treatment of fungal over-infections of the oral cavity and genital area, the treatment of itchy scratching lesions in demented patients, the cleansing of bedsores which in a few days clearly reduces the malodorous aspect, when present, the treatment of diabetic sores with undermined infected lesions, the cleansing of varicose ulcers, the treatment of first degree burns.

The therapeutic effects have also proven effective in the veterinary field.

The present invention can be better understood in the light of the following embodiments.

### BRIEF DESCRIPTION OF THE FIGURES

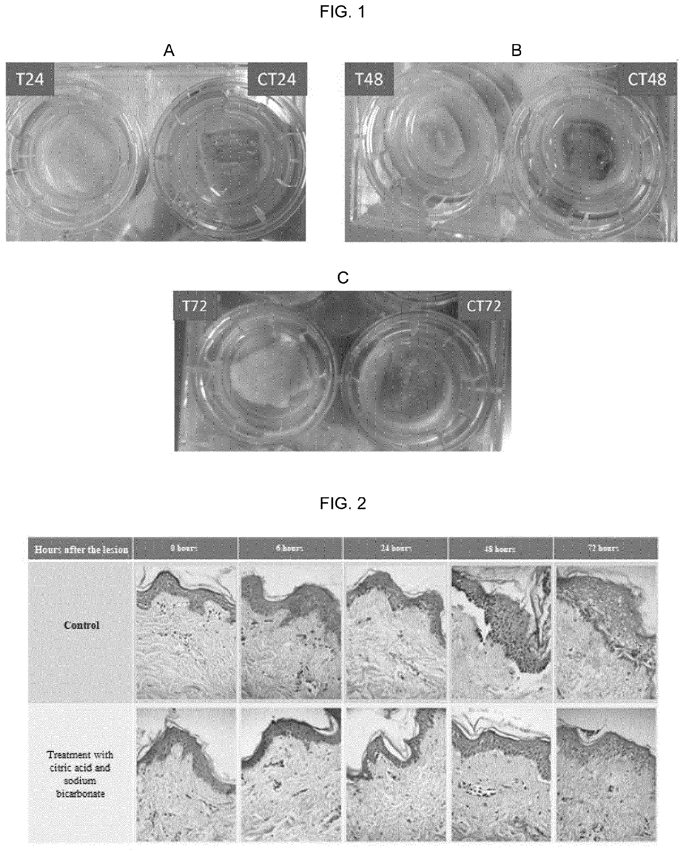

Fig. 1 shows skin samples cultured after skin damage; A: shows the samples after 24h, treated with the composition of the invention (T24) and untreated (CT24); B: shows the samples after 48h, treated with the composition of the invention (T48) and untreated (CT48); C: shows the samples after 72h, treated with the composition of the invention (T72) and untreated (CT72); where it is clearly seen that *untreated skin samples show signs of skin intolerance and inflammation.*
Fig. 2 shows a photo.

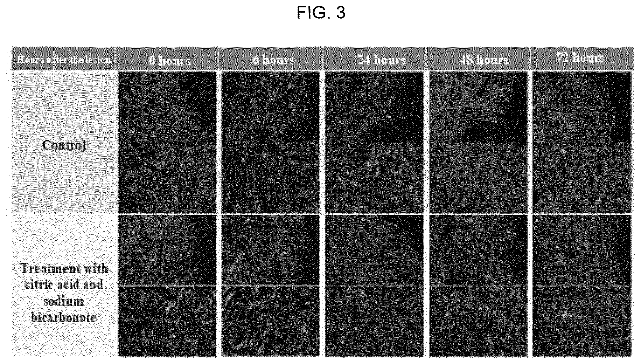

Fig. 3 shows a photo. Both in the control and in the treatment photos, the upper half indicates the Papillary Dermis, the lower half the Reticular Dermis

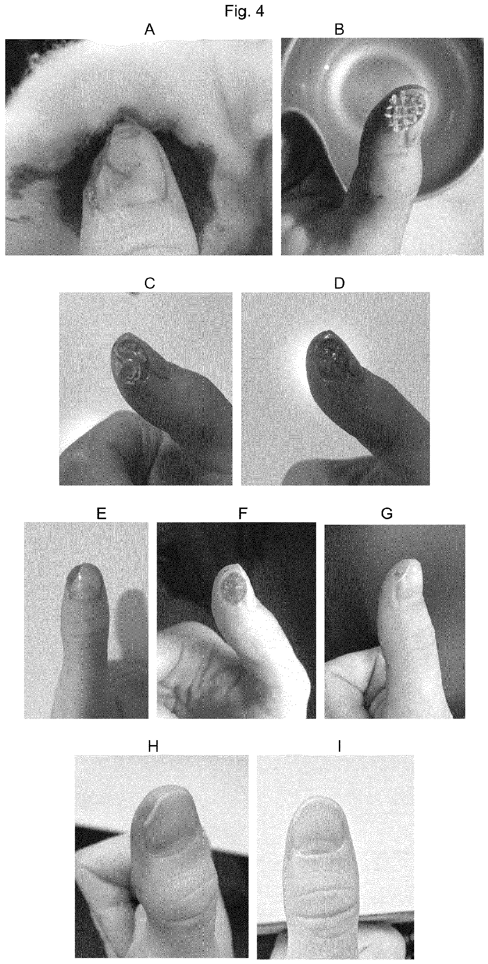

Fig. 4 shows the healing of an accidental cut injury by a mandoline slicer with removal of a corner of the thumb nail phalanx. A: lesion on Day 0; lesion on Day 2 with connettivina gauze applied in the emergency room on the day of the injury; B: lesion on Day 3 after compresses with the aqueous composition of Citric acid + NaHCOs of the invention and subsequent application of Kaqun+HA+D-panthenol gel (3 times/day); C: lesion on Day 4; D: lesion on Day 5; E: lesion on Day 7, less painful wound; F: lesion on Day 14; G. lesion on Day 23; H: lesion on Day 31; I: lesion on Day 51, perfectly healed.

### EXPERIMENTAL PART

### EXAMPLE 1 - Clinical study of skin lesions treatment

To verify the efficacy of the mixtures developed, a group of 88 patients was treated, the patients being affected by acute skin lesions resulting from simple skin abrasions in children, cleansing of acute and sub-acute radiodermatitis, oral cavity, genital and anal mucositis, for the treatment of fungal over-infections of the oral cavity and genital area, the treatment of itchy scratching lesions in demented patients, the cleansing of bedsores, the treatment of diabetic sores with undermined infected lesions, the cleansing of varicose ulcers, the treatment of first degree burns.

For the evaluation of erythematous skin lesions, we adopted the criteria of Pathak et al. (Preventive treatment of sunburn, dermatoheliosis and skin cancer with agents. Dermatology in general medicine, 3rd, Ed. Fitzpatrick TB, Eds New York, Mc Graw Hill; 1987, 1507-1592)

The lesions found in the oncological environment ranged from simple erythematous radiodermatitis, to epidermolysis, to erythemato-bullous radiodermatitis, in the range of Grades 2 (45 cases), 3 (31 cases) and 4 (12 cases)

In the presence of Grade 2, 3 and 4 lesions, the treatment adopted consisted of: a) rinsing with aqueous solution of Citric Acid Monohydrate + Sodium Bicarbonate (three times a day); b) placing of the gel with water enriched in oxygen, D-Panthenol and Hyaluronic acid after about 30 minutes.

All the patients observed by us declared that they had obtained immediate benefits from the therapies carried out and were satisfied.

For simplicity, safety and efficacy, the therapeutic combination disclosed consisting of sterile soaked gauze compresses for 20-30' and daily rinsing with aqueous solution of Citric Acid Monohydrate + Sodium Bicarbonate can represent, according to our results, a valid therapeutic solution to be strongly recommended in similar cases, especially for its ability to thoroughly cleanse the wound, eliminating fibrin and promoting the healing process.

### EXAMPLE 2 - In vitro study of skin damage

After having caused skin damage, we wanted to evaluate whether the solution of citric acid and sodium bicarbonate was able to activate tissue repair mechanisms in the cultured skin, within a short time.

The results were surprising as can be seen from Fig. 1 where it is clearly seen that *untreated skin samples show signs of skin intolerance and inflammation.*

The treatment favors the preservation of the normal terminal differentiation and integrity of the epidermis, together with the physiological epithelial-mesenchymal interactions (Fig. 2).

The treatment favors a more rapid collagen neo-deposition and the remodeling of the extracellular matrix when compared to the control (Fig. 3).

### EXAMPLE 3 - In vitro study of antibacterial activity against SA and MRSA

A microbiological experimentation was performed to verify whether the aqueous composition of the invention, or single components thereof, were capable of inhibiting bacterial growth *in vitro,* and in particular of *Staphylococcus aureus* (SA) and methicillin resistant *S*. *aureus* (MRSA), also considering the prevalence of the methicillin resistance factor in Italy.

The procedure normally adopted for MICs determination was followed, therefore bacterial suspensions of both MSSA and MRSA S. *aureus* were prepared in a concentration equal to 10⁷.

The solution whose antibacterial capacity had to be evaluated was also tested in parallel on bacterial strains resistant to vancomycin, linezolid and daptomycin.

All substances were tested as follows:
- a drop equal to 50 µl of substance was placed in contact with the surface of Mueller-Hinton agar plates inoculated with the bacterial suspensions;
- pieces of sterile gauze about one centimeter thick, impregnated with the substance, were placed in contact with the surfaces of the plates inoculated with the bacteria;
- one cc of the substances was placed in contact with an equal amount of bacterial suspension, 100 µl of the same were seeded in the plate after 1 hour and after 24 hours;
- 100 µl of the substances were placed in direct contact with the plates in the plateau phase and not in the logarithmic growth phase.

All the substances, with the exception of the Citric Acid + Sodium Bicarbonate solution, have shown little or no ability to inhibit the bacteria with which they were placed in contact, neither in the logarithmic growth phase nor in the plateau phase, except for a slight inhibition of the culture gel both after one hour and after 24 hours of contact. But above all, the solution used in contact with the plate surface showed a remarkable activity against *S*. *aureus.* The comparison with a traditional antibiotic also showed that the activity of the solution is comparable to that of vancomycin. The fact that appears evident is that the solution examined certainly does not behave as an antibiotic nor probably as a disinfectant, but certainly has a bacteriostatic action against *S*. *aureus.*

### EXAMPLE 4 - In vitro studies of antimicrobial activity against various pathogenic microorganisms

The purpose of the study is to demonstrate the bactericidal capacity of a CITRIC ACID and SODIUM BICARBONATE MIXTURE in aqueous solution.

### Material:

- CITRIC ACID (45.95% w/w) and SODIUM BICARBONATE (54.05% w/w) MIXTURE

- Saline in sterile containers
- Mannitol Salt Agar Biomerieux plates; this medium is used because the colonies of *Staphylococcus aureus* turn yellow on the plate
- 100 µl pipette with sterile tips
- Sterile L-shaped spatula

### Method:

A suspension is prepared for each of the bacteria in question in Brain Heart Broth incubating them for 48 hours.

The titer of the bacterial suspension is checked with a turbidimeter or by diluting it 1:100 to reach a turbidity of 0.5 MF, which approximately corresponds to a concentration of 1×10⁸ CFU/ml. This represents the titer that will be used as inoculum.

5 ml of sterile saline are introduced into three 50 ml-tubes, one for each bacterial strain, one tube for the blank.

3.5 g - 7.0 g - 14 g of CITRIC ACID and SODIUM BICARBONATE MIXTURE will be added, respectively.

The end of the effervescent reaction and the solubilization of all the product are allowed to happen. 50 µl of the bacterial culture are introduced into these three test tubes obtaining a 1/100 dilution in the test tubes we will have about 1×10⁶ CFU/ml of the strains under test.

At time 0, the inoculated load was determined by spreading about 100 µl of scalar dilutions of the blank on the respective selective plates and incubating at 37°C for 24 hours.

All tubes are incubated at 37°C for 24 hours.

After 24 hours, 100 µl of each single tube are spread on the respective selective media, including the blank, and incubated at 37°C for 24 hours.

### Results:

### Klebsiella

| | | | | |
|---|---|---|---|---|
| **Time** | **Blank tube** | **3.5 g** | **7.0 g** | **14 g** |
| Zero | ≃ 10⁶ | // | // | // |
| After 24 hours | ≃ 10⁶ | 2.7 x 10³ | <100 | <100 |
| **% abatement** | **0** | **99.73** | **99.99** | **99.99** |

### E. coli

| | | | | |
|---|---|---|---|---|
| **Time** | **Blank tube** | **3.5 g** | **7.0 g** | **14 g** |
| Zero | ≃ 10⁶ | // | // | // |
| After 24 hours | ≃ 10⁶ | 3.1 x 10³ | <100 | <100 |
| **% abatement** | **0** | **99.69** | **99.99** | **99.99** |

### Staphylococcus aureus

| | | | | |
|---|---|---|---|---|
| **Time** | **Blank tube inoculum** | **3.5 g** | **7.0 g** | **14 g** |
| Zero | ≃ 10⁶ | // | // | // |
| After 24 hours | ≃ 10⁶ | >10⁵ | >10⁵ | 2.2 x 10² |
| **% abatement** | **0** | **0** | **0** | **98.78** |

### Candida

| **Time** | **Blank tube** | **3.5 g** | **7.0 g** | **14 g** |
|---|---|---|---|---|
| Zero | ≃ 10⁶ | // | // | // |
| After 24 hours | ≃ 10⁶ | <100 | <100 | <100 |
| **% abatement** | **0** | **99.99** | **99.99** | **99.99** |

### Pseudomonas aeruginosa

| **Time** | **Blank tube inoculum** | **3.5 g** | **7.0 g** | **14 g** |
|---|---|---|---|---|
| Zero | ≃ 10⁶ | // | // | // |
| After 24 hours | ≃ 10⁶ | 3.4 × 10² | <100 | <100 |
| **% abatement** | **0** | **99.60** | **99.99** | **99.99** |

For *Klebsiella, E. coli, P. aeruginosa* and *Candida* the bactericidal activity is already seen at the concentration of 3.5g/5ml of CITRIC ACID and SODIUM BICARBONATE MIXTURE.

For *Staphylococcus aureus,* the bactericidal activity is seen at a concentration of 14g/5ml of CITRIC ACID and SODIUM BICARBONATE MIXTURE.

### EXAMPLE 5 - Treatment of lesions infected with Candida Albicans

We extended the application of the Citric Acid and Sodium Bicarbonate solution to infected wounds and ascertained its efficacy also in cleansing wounds infected with *Candida Albicans.*

In this sense, we obtained healings of vaginal Candida infections through the use of douches.

A few days after the surgery, a patient who had just undergone a left mastectomy with immediate breast reconstruction experienced a breast infection which in about ten days resulted in a fistula. Cleaning of the fistula and immediately performed antibiotic therapies did not solve the problem. An antibiogram of the fistulous secretion was performed, which documented an infection from *CANDIDA ALBICANS.* The surgical team, after various treatment attempts with systemic antifungals, was ready for surgery to remove the prosthesis and cleanse the wound.

The patient who did not want to undergo surgery, went elsewhere and immediately began to treat herself with compresses and washings of the solution object of the present invention until the fistula remained open, attempting to spray the solution directly into the lesion using a needleless syringe.

In a few days she noticed a clear reduction in the infection, and in about ten days the fistula had closed.

### EXAMPLE 6 - Treatment of canker sores and fungal infections of the oral cavity

We also achieved resolution of fungal infections of the oral cavity and canker sores. In this case we have used both rinses with solutions according to the invention. This second method of application has proved to be effective for the solution of canker sores. Repeating the application 3/4 times a day is enough to obtain healing in 2, maximum 3 days.

## Claims

1. An aqueous solution consisting of water in which a mixture of citric acid and sodium bicarbonate has been dissolved, said aqueous solution for topical use as an antimicrobial and/or in the topical treatment of acute and/or chronic skin and/or mucous membranes lesions, including infected ones.

2. The solution according to claim 1, wherein the mixture of citric acid and sodium bicarbonate consists of:
| | |
|---|---|
| Citric acid | 40-50% w/w; |
| NaHCO₃ | 50-60% w/w. |

3. The solution according to any one of claims 1-2, wherein the mixture of citric acid and sodium bicarbonate is dissolved in an adequate amount of water so as to obtain an aqueous solution comprising:
| | |
|---|---|
| Citric acid | 5-35% w/w, preferably 5-20% w/w; |
| NaHCO₃ | 5-40% w/w, preferably 5-22% w/w. |

4. The solution according to any one of claims 1-3 further comprising a hemostatic agent.

5. The solution according to claim 4, wherein the hemostatic agent is contained at 2-15% w/w and is a combination of iron ammonium citrate (FAC) and potassium alum in a weight ratio of 4:1-2:1.

6. The solution according to any one of claims 1-5 further comprising a moisturizing and/or antioxidant and/or antimicrobial agent.

7. The solution according to claim 6, wherein the moisturizing and/or antioxidant and/or antimicrobial agent is contained at 0.2-2% w/w and is a mixture of 1,2-hexandiol, 1,2-octandiol and Tropolone.

8. A kit for sequential use in the topical treatment of acute and/or chronic skin and/or mucous membranes lesions, including infected ones, said kit comprising:
a solution according to any one of claims 1-7, to be applied first;
a gel based on water enriched in oxygen, D-Panthenol and hyaluronic acid, to be applied subsequently.

9. The kit according to claim 8, wherein the gel comprises:
| | |
|---|---|
| D-panthenol | 1-9%, preferably 4-6%; |
| sodium hyaluronate | 0.05-4%, preferably 0.1-0.3%; |
| Kaqun water | balance to 100%; |
where the percentages are % w/w referred to the total weight of the composition.

10. The solution according to any one of claims 1-7 or a kit according to any one of claims 8-9 for topical use as an antimicrobial on skin and/or mucous membranes lesions infected with *Klebsiella, E. coli, Candida Albicans, Staphylococcus aureus* and *Pseudomonas aeruginosa.*

11. The solution according to any one of claims 1-7 or a kit according to any one of claims 8-9 for use in the topical treatment of acute skin and/or mucosal lesions, including infected ones, in particular if consequent to radiotherapy, bedsore, diabetic foot and wounds from a traumatic event, simple skin abrasions, cleansing of acute and sub-acute radiodermatitis, oral cavity, genital and anal mucositis, for the treatment of fungal over-infections of the oral cavity and genital area, the treatment of itchy scratching lesions in demented patients, the cleansing of bedsores, the treatment of diabetic sores with undermined infected lesions, the cleansing of varicose ulcers, the treatment of first degree burns.
